# FASCICULE DE BREVET EUROPEEN

(11) **EP 0 748 482 B1**
(45) Date de publication et mention de la délivrance du brevet: **03.02.1999**
(21) Numéro de dépôt: 95908180.3
(22) Date de dépôt: 23.02.1995
(51) Int. Cl.: G06F 19/00

(54) **SYSTEME MEDICAL D'AIDE A LA DECISION ET DISPOSITIF D'ADMINISTRATION D'AU MOINS UN MEDICAMENT**
MEDIZINISCHES ENTSCHEIDUNGSUNTERSTÜTZUNGSSYSTEM UND VORRICHTUNG ZUR VERABREICHUNG MINDESTENS EINES HEILMITTELS
MEDICAL DECISION-MAKING AID AND DEVICE FOR DELIVERING AT LEAST ONE DRUG

(30) Priorité: 04.03.1994 CH 644/94
(43) Date de publication de la demande: 18.12.1996
(73) Titulaire: Microflow Engineering SA, 2007 Neuchâtel (CH)
(72) Inventeur: HESS, Joseph, CH-2022 Bevaix (CH)
(74) Mandataire: Patry, Didier Marcel Pierre
(86) Numéro de dépôt international: CH9500038
(87) Numéro de publication internationale: WO9524013

(56) Documents cités:
- US-A- 5 153 827
- PROCEEDINGS OF ANNUAL INTERNATIONAL CONFERENCE OF THE IEEE/EMBS, IEEE PRESS NEW YORK US, vol. 11, SEATTLE, US, page 260 D.G.MASON ET AL 'CLOSED LOOP CONTROL OF MULTIPLE DRUG INFUSION'
- IEEE TRANSACTIONS ON BIOMEDICAL ENGINEERING, IEEE PRESS, vol. 39, NEW YORK, page 1060 HAO YING ET AL 'FUZZY CONTROL OF MEAN ARTERIAL PRESSURE IN POSTSURGICAL PATIENTS WITH SODIUM NITROPRUSSIDE INFUSION'
- COMPUTING & CONTROL ENGINEERING JOURNAL, vol. 4, UK, page 55 D.A.LINKENS ET AL 'ANAESTHESIA SIMULATORS FOR THE DESIGN OF A SUPERVISORY RULE-BASED CONTROL IN THE OPERATING THEATRE'
- MICROCOMPUTER APPLICATIONS, vol. 6, US, page 16 C.HERNANDEZ ET AL 'COMPUTERIZED CLOSED LOOP CONTROL OF PREOPERATIVE ANTI-HYPERTENSIVE ADMINISTRATION'
- INTERNATIONAL JOURNAL OF CLINICAL MONITORING AND COMPUTING, vol. 8, NL, page 25 J.A.BLOM 'EXPERT CONTROL OF THE ARTERIAL BLOOD PRESSURE DURING SURGERY'
- ANNALS OF BIOMEDICAL ENGINEERING, vol. 13, US, page 217 B.C.MCINNIS ET AL 'AUTOMATIC CONTROL OF BLOOD PRESSURES WITH MULTIPLE DRUG INPUTS'
- IEEE TRANSACTIONS ON BIOMEDICAL ENGINEERING, vol. 34, US, page 633 C.YURKONIS ET AL 'COMPUTER SIMULATION OF ADAPTIVE DRUG INFUSION'
- JOURNAL OF INTELLIGENT AND FUZZY SYSTEMS, vol.1, no.1, , page 27 - 42, MIKI ET AL. 'SILICON IMPLEMENTATION FOR A NOVEL HIGH-SPEED FUZZY INFERENCE ENGINE'

## Description

La présente invention concerne le domaine de l'administration de médicament(s) à un être vivant, en particulier à un patient subissant un traitement thérapeutique.

Il est connu du document intitulé "Anaesthesia simulators for the design of supervisory rule-based control in the operating theatre" de D.-A. Linkens et M. F. Abbod, Compating & Control Engineering J., April 93, v. 4, No 2, pages 55 to 62, un circuit de commande à logique floue pour l'administration en temps réel d'un anesthésiant lors d'une opération qui comprend un asservissement à circuit fermé et un système de supervision du type "expert" permettant une évolution et une adaptation des algorithmes à logique floue et de facteurs d'échelle en entrée et en sortie du module de traitement à logique floue par le système de supervision lui-même.

Ce système de commande inclut des moyens permettant d'introduire la concentration du médicament à administrer et des informations relatives au patient. Il fournit en sortie un signal électrique de commande à un dispositif d'administration du médicament, ce signal électrique correspondant à un débit instantané dudit médicament et variant en fonction dudit asservissement qui tient compte de la réponse en temps réel du patient par l'intermédiaire d'un relaxographe. Le système asservi comprend des moyens aptes à faire varier des facteurs d'échelle d'entrée et de sortie respectivement de part et d'autre du circuit de commande à logique floue.

Pour ce qui concerne la détermination par un médecin-praticien d'une ou plusieurs dose(s) de médicament à administrer à un patient, elle est généralement effectuée sur la base de doses standards données par le fabricant du médicament. Dans certains cas, il est donné une plage de valeurs possibles pour chaque dose et un nombre standard d'administrations journalier de cette dose. Sur cette base, le médecin-praticien prescrit une certaine dose suite à son diagnostic qui établit la gravité de la maladie ou du problème physique ou moral de son patient. Cette prescription repose donc sur l'expérience du médecin-praticien et sur son appréciation de la maladie ou du problème constaté.

Le médecin-praticien est donc confronté à un problème de détermination de doses de médicament(s) pour différents patients sur la seule base de données générales en la matière, ce problème découlant notamment du fait qu'une personnalisation de ces doses dépend d'un ensemble de paramètres déterminés que le médecin-praticien ne peut pas gérer dans la pratique d'une manière sûre et efficace.

Un premier but de l'invention est de fournir un système d'aide à la décision servant à déterminer une quantité personnalisée d'au moins un médicament donné à administrer à un être vivant suite à un diagnostic effectué par un médecin-praticien.

A cet effet, l'invention concerne un système d'aide à la décision pour l'administration d'au moins un médicament à un être vivant tel que revendiqué à la revendication 1. Les informations relatives au(x) médicament(s) peuvent être fournies par l'utilisateur ou introduites au préalable par le fournisseur du système d'aide à la décision.

L'utilisation d'une unité de traitement à logique floue permet d'introduire les informations sous forme de variables floues exprimées par exemple à l'aide d'une appréciation qualitative. Par appréciation qualitative, il est compris une appréciation exprimée sur une échelle décrivant divers états possibles pour un paramètre donné.

Le traitement des divers paramètres par une unité de traitement à logique floue est très efficace pour une telle application et ne nécessite pas une complexité particulière pour ladite unité de traitement, ce qui la rend relativement peu onéreuse. De plus, un tel traitement n'est généralement pas à la portée des facultés intellectuelles d'un médecin-praticien de telle sorte que le système d'aide à la décision selon l'invention joue pleinement un rôle d'aide à la détermination de doses personnalisées de médicament(s); ceci d'autant plus que le nombre de paramètres à gérer est élevé. L'utilisation de la logique floue dans le système de l'invention permet donc de tirer profit de manière sûre et efficace des connaissances acquises dans les domaines de la médecine et de la pharmacie pour déterminer des doses personnalisées optimales de médicament(s) avec une précision suffisante. Les valeurs physiologiques d'un être vivant étant généralement comprises dans des plages de valeurs et n'étant pas constantes dans le temps, un traitement par logique floue est très approprié.

Depuis l'émergence de la théorie de la logique floue dans les années 1960, d'innombrables articles, livres et autres documents ont été publiés sur cette théorie et ses applications (voir par exemple L.A. Zadeh dans Information sciences 8, p. 199-249, 1975, et les nombreuses références citées dans cet article).

Pour ce qui concerne l'intégration des diverses règles d'inférence et algorithmes de traitement de données à logique floue dans des circuits électriques ou électroniques, l'homme du métier dispose actuellement d'une connaissance étendue en la matière. De nombreux articles, livres et autres documents ont été publiés à ce sujet outre le dernier document cité ci-avant, par exemple T. Yamakawa and T. Miki, The Current Mode Fuzzy Logic Integrated Circuits Fabricated by the Standard CMOS Process, IEEE Transactions on computers, vol. C-35, No 2, p. 161 à 167, February 1986; K. Bult and H. Wallinga, A Class of Analog CMOS Circuits Based on the Square-Law Characteristic of an MOS Transistor in Saturation, IEEE Journal of Solid State Circuits, vol. SC-22, No 3, p. 357 à 365, June 1987; T. Miki & al., Silicon implementation for a novel high-speed fuzzy inference Engine : Mega-Flips Analog Fuzzy Processor, Journal of Intelligent and Fuzzy Systems, vol. 1(1), p. 27-42, 1993; et en particulier M. Sasaki et al., Current-Mode Analog Fuzzy Hardware with Voltage Input Interface and Normalization Locked Loop, IEICE Trans. Fundamentals. Vol. E75-A No 6, p. 650 à 654, June 1992 et Proc. 1st IEEE Conference Fuzzy Systems, San Diego, p. 451 à 457, March 1992; M. Sasaki & al., Fuzzy Multiple-Input Maximum and Minimum Circuits in Current Mode and Their Analyses Using Bounded-Difference Equations, IEEE Transactions on Computers, Vol. 39, No 6, p. 768-774, June 1990.

La création d'algorithmes à logique floue, une fois les divers paramètres et variables données, et la conception de circuits pour leur exécution, notamment sous forme de circuits primaires pour les règles d'inférence et de circuits d'aggrégation des diverses règles d'inférence, faisant partie du travail habituel de l'homme du métier, elles ne seront pas par la suite décrites en détail.

La présente invention porte sur un nouveau type de système d'aide à la décision pour le domaine médical qui est basé sur un concept nouveau permettant au médecin-praticien de personnaliser efficacement le dosage de médicament(s) pour un patient particulier et une thérapie particulière tout en restant applicable à d'autres patients. Par exemple, des microprocesseurs du genre FP 9000 et FP 9001 de la société OMRON, Kyoto, Japan, dont il est fait mention dans l'article de T. Miki & al. (pages 38 à 40) cité ci-avant, peuvent être utilisés dans une réalisation d'un système d'aide à la décision selon la présente invention, tout comme des circuits de type "current mode" similaires à ceux proposés par M. Sasaki & al. ou K. Bult and H. Wallinga cités ci-avant qui permettent un traitement analogique des divers paramètres et variables.

Le système d'aide à la décision selon l'invention peut être agencé pour traiter en entrée plusieurs informations relatives aux médicaments et plusieurs informations relatives à l'être vivant auquel ce médicament est destiné.

Pour ce qui concerne les informations relatives aux médicaments, ils comprennent par exemple la valeur de la dose standard donnée par le fabricant du médicament, le nombre d'administrations journalier de cette dose, le temps ou la période d'administration de chaque dose au cours de la journée ou/et un intervalle de temps entre deux doses consécutives. Ces informations peuvent concerner aussi l'organe auquel est destiné le médicament, le débit maximum pour l'administration de ce médicament et/ou alors la sélection d'une fonction temporelle préprogrammée du débit au cours de l'administration d'une dose. En termes plus généraux, les informations liées à un médicament concernent des données propres à ce médicament, aux organes ou parties du corps concernées par ce médicament et au mode d'administration dans le temps de ce médicament.

Pour ce qui concerne les informations relatives à l'être vivant auquel le médicament est destiné, ils comprennent par exemple des données comme l'âge, le poids, les habitudes relatives à la fumée et à la consommation d'alcool, les allergies, ainsi que des données résultantes d'un diagnostic comme la pression, le rythme cardiaque, la température, la concentration d'une substance dans le sang.

Toutes les informations relatives aux médicaments et à l'être vivant auquel il est destiné sont traitées par l'unité de traitement à logique floue par le moyen de diverses règles d'inférence servant à définir une quantité personnalisée du médicament et un mode d'administration personnalisé de ce médicament dans le temps.

Dans un premier mode de réalisation, l'unité de traitement à logique floue fournit en sortie un seul signal représentatif d'une quantité de médicament personnalisée à administrer. Cette quantité peut concerner la dose à donner chaque fois que le médicament est administré ou alors une quantité à donner à l'être vivant auquel le médicament est destiné sur une certaine période.

Dans un deuxième mode de réalisation de l'invention, l'unité de traitement à logique floue fournit en outre un deuxième paramètre relatif au mode d'administration dans le temps du médicament.

Selon d'autres caractéristiques particulières de l'invention, le système d'aide à la décision est agencé pour traiter des données relatives à plusieurs médicaments différents devant être administrés au même être vivant. Dans un tel cas, l'unité de traitement à logique floue peut être agencée de manière que divers effets entre les médicaments prévus soient pris en considération dans le dosage de chaque médicament et dans le mode d'administration dans le temps de chacun de ces médicaments.

Selon d'autres caractéristiques particulières de l'invention, le système d'aide à la décision est agencé pour traiter des premières informations relatives aux médicaments et à l'être vivant auquel il est destiné et conjointement des deuxièmes informations relatives aux degrés de pertinence des premières informations. Ces degrés de pertinence forment des facteurs de pondération liés respectivement aux premières informations. Ces facteurs de pondération sont pris en considération dans le traitement des données par l'unité de traitement à logique floue et peuvent intervenir dans le calcul du degré de compatibilité ou de satisfaction des diverses règles d'inférence prévues dans le traitement des informations fournies à l'unité de traitement à logique floue et/ou lors de l'agrégation des résultats de ces diverses règles d'inférence. Cette agrégation des résultats peut par exemple être réalisée, dans une étape particulière du processus, par une union pondérée desdits résultats.

Grâce aux possibilités très étendues de la logique floue, le système d'aide à la décision selon l'invention peut être très performant et intégrer plusieurs informations d'entrée tout en restant relativement simple dans sa structure et peu onéreux dans sa réalisation.

Selon une autre caractéristique particulière de l'invention, l'unité de traitement à logique floue peut être associée à une base de données dans laquelle peuvent être stockées une quantité d'informations concernant les divers organes, les divers médicaments, ainsi que des données relatives à un dispositif d'administration prévu pour l'administration du ou des médicament(s).

La présente invention a encore comme but de fournir un dispositif d'administration de médicaments permettant une administration personnalisée d'au moins un médicament.

A cet effet, la présente invention concerne un dispositif d'administration d'au moins un médicament fluide à un être vivant comprenant au moins une pompe, externe ou implantée, pour l'administration dudit médicament et un circuit de commande de cette pompe, ce dispositif d'administration étant caractérisé en ce qu'il comprend en outre un système d'aide à la décision selon l'invention fournissant ledit signal de sortie audit circuit de commande.

Le dispositif d'administration selon l'invention permet ainsi de déterminer une quantité personnalisée du médicament fluide prévu et de l'administrer de manière automatique et autonome. Selon un mode de réalisation préféré, le système d'aide à la décision fournit au circuit de commande au moins une information relative à une quantité personnalisée du médicament fluide à administrer à l'être vivant auquel il est destiné et au moins une information relative à un mode d'administration personnalisé dans le temps de ce médicament fluide à cet être vivant.

Grâce à ces caractéristiques, il est possible d'administrer de manière autonome et personnalisée au cours du temps un médicament à un être vivant.

Selon un mode de réalisation particulier, le dispositif d'administration est agencé pour administrer sélectivement plusieurs médicaments au moyen de ladite pompe à un être vivant.

Par exemple, la pompe peut être constituée par une micropompe actionnée par un élément piézo-électrique, un actionnement rotatif du type péristaltique, un actionnement linéaire du type pousse-seringue ou une configuration multiple de ces actionnements.

D'autres caractéristiques et avantages de l'invention seront également décrits à l'aide de la description suivante, faite en référence aux dessins annexés, donnés à titre d'exemples nullement limitatifs, sur lesquels :
- la figure 1 représente schématiquement un premier mode de réalisation de l'invention;
- la figure 2 représente schématiquement un deuxième mode de réalisation de l'invention.

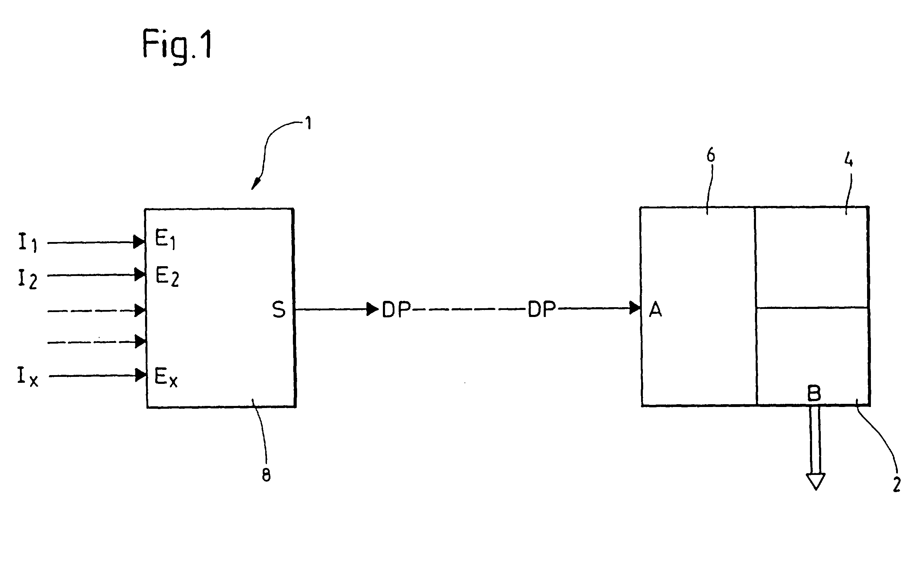

A l'aide de la figure 1, on décrira ci-après un premier mode de réalisation de l'invention.

Sur cette figure 1 est représenté schématiquement d'une part un système d'aide à la décision 1 et d'autre part une pompe 2 servant à l'administration d'un médicament fluide, cette pompe étant associée à un réservoir 4 et à un circuit de commande 6.

Le système d'aide à la décision 1 comprend une unité de traitement à logique floue 8 comprenant x entrées E₁, E₂, ..., Eₓ et une sortie S. Chacune des entrées E₁ à Eₓ sont agencées pour recevoir respectivement x paramètres ou informations I₁, I₂, ..., Iₓ.

Selon l'invention, au moins une des informations I₁ à Iₓ est relative à un médicament destiné à être administré à un être vivant et au moins une autre information est relative à cet être vivant.

Par être vivant, il est compris essentiellement des personnes humaines ou des animaux.

L'unité de traitement à logique floue 8 est agencée pour fournir un signal de sortie DP représentatif d'une quantité personnalisée du médicament à administrer à l'être vivant auquel il est destiné. Le signal de sortie DP peut définir une dose personnalisée précise ou une dose donnée par une consigne floue qui sera traitée ultérieurement par un médecin ou par le circuit de commande 6.

L'unité de traitement à logique floue 8 est agencée pour traiter de manière floue les diverses informations Il à Iₓ fournies au système d'aide à la décision 1 à l'aide de diverses règles d'inférence prédéterminées avant l'introduction des informations I₁ à Iₓ. Les résultats donnés par ces règles d'inférence sont traitées par l'unité de traitement à logique floue 8 qui fournit alors à sa sortie S un signal de sortie DP dont la valeur dépend des informations I₁ à Iₓ fournit en entrée.

On notera ici que les diverses règles d'inférence et la manière dont les résultats obtenus par ces règles d'inférence sont agglomérés ensemble peuvent être intégrées dans ladite unité de traitement à logique floue 8 de manière définitive ou, dans un mode de réalisation plus évolué, être au moins introduite partiellement à l'aide d'une interface (non-représenté) permettant d'introduire diverses règles d'inférence ou au moins de varier certains paramètres de traitement de ces diverses règles d'inférence. Dans ce dernier mode de réalisation particulier de l'invention, le système d'aide à la décision 1 peut répondre à diverses situations différentes et être adaptées à l'administration de divers médicaments de différentes natures avec un haut degré d'efficacité.

Il est possible d'envisager que le fabricant de chaque médicament est à même de fournir un ensemble de données permettant de programmer l'unité de traitement à logique floue 8 par l'intermédiaire dudit interface avant que le système d'aide à la décision 1 soit utilisé pour la détermination d'une dose ou d'une quantité personnalisée de ce médicament à administrer à un être vivant déterminé. Le fabricant de médicaments fournira par exemple un code-barres ou une carte électronique du type carte à puce, du type PCMCIA ou du type commande/transmission à distance. Le système d'aide à la décision comprend des moyens pour lire et interpréter les informations contenues dans le code barres ou dans les cartes électroniques à puce, PCMCIA ou de commande/transmission à distance. Ces informations peuvent être codées par le fabricant de médicaments et le dispositif d'administration est alors agencé pour lire un tel code, ce dispositif ne fonctionnant que pour un ensemble de médicaments prédéterminés. Ces informations pourront en outre être utilisées dans des logiciels pour des micro-ordinateurs portables ou de bureau pour la formation du personnel traitant et/ou le suivi des thérapies. Le système d'aide à la décision comprend des moyens pour lire les informations contenues dans le code-barres ou dans la carte électronique.

Les informations I₁ à Iₓ peuvent être formées de plusieurs informations relatives au médicament et de plusieurs informations relatives à l'être vivant auquel le médicament est destiné. A titre d'exemple, les informations relatives au médicament comprennent la valeur d'une dose standard ou d'une quantité du médicament standard à administrer durant une certaine période, cette dose standard ou cette quantité standard étant généralement fournie par le fabricant du médicament, l'organe ou les organes auxquels sont destinés en particulier le médicament, la composition chimique du médicament ou toute autre information susceptible d'influencer la quantité de médicament à administrer à un être vivant déterminé.

Concernant les informations relatives à l'être vivant auquel le médicament est destiné, elles peuvent être très diverses et comprennent par exemple des données relatives à l'âge, le poids, les habitudes de vie, les allergies de cet être vivant, ainsi que des données résultantes d'un diagnostic comme la pression, la température, la concentration d'une substance dans le sang, un organe ou une partie du corps atteinte par une maladie, le nom de cette maladie et toute autre information propre à influencer la quantité de médicament à administrer à cet être vivant.

Le système d'aide à la décision 1 selon l'invention permet donc de traiter diverses informations relatives à un médicament et à un être vivant auquel ce médicament est destiné de manière à fournir une quantité individualisée de ce médicament à fournir à cet être vivant. Ce système d'aide à la décision 1 permet ainsi d'établir une thérapie personnalisée pour un certain médicament sur la base d'informations diverses, en particulier d'informations qualitatives concernant l'être vivant soumis à ladite thérapie. De ce fait, la thérapie est rendue plus efficace, les risques secondaires liés au médicament sont minimisés.

On notera ici que le système d'aide à la décision 1 peut former un système autonome comprenant des moyens de lecture permettant de lire le signal de sortie DP ou être combinés avec un dispositif d'administration du médicament, en particulier avec une pompe d'administration d'un médicament fluide.

Il est possible de prévoir à l'entrée A du circuit de commande 6 de la pompe 2 une interface de lecture (non-représenté) agencé pour recevoir ledit signal de sortie DP directement du système d'aide à la décision 1, l'information donné par ledit signal de sortie DP étant ensuite stockée dans le circuit de commande 6, ce circuit de commande 6 étant alors séparé du système d'aide à la décision 1 lors de l'administration du médicament à l'être vivant auquel il est destiné.

Ainsi, le système d'aide à la décision 1 peut être entièrement autonome et comprendre son propre dispositif de lecture ou être autonome dans son fonctionnement tout en comprenant une interface adapté à une interface de lecture prévu en entrée du circuit de commande 6 de la pompe 2. Dans un mode de réalisation intégré, le système d'aide à la décision 1 peut être associé au circuit de commande 6 de manière à former ensemble un seul module électronique.

Sur la base de l'information reçue à l'entrée A du circuit de commande 6, la pompe 2 fournit à sa sortie B une quantité de médicament correspondant au signal de sortie DP.

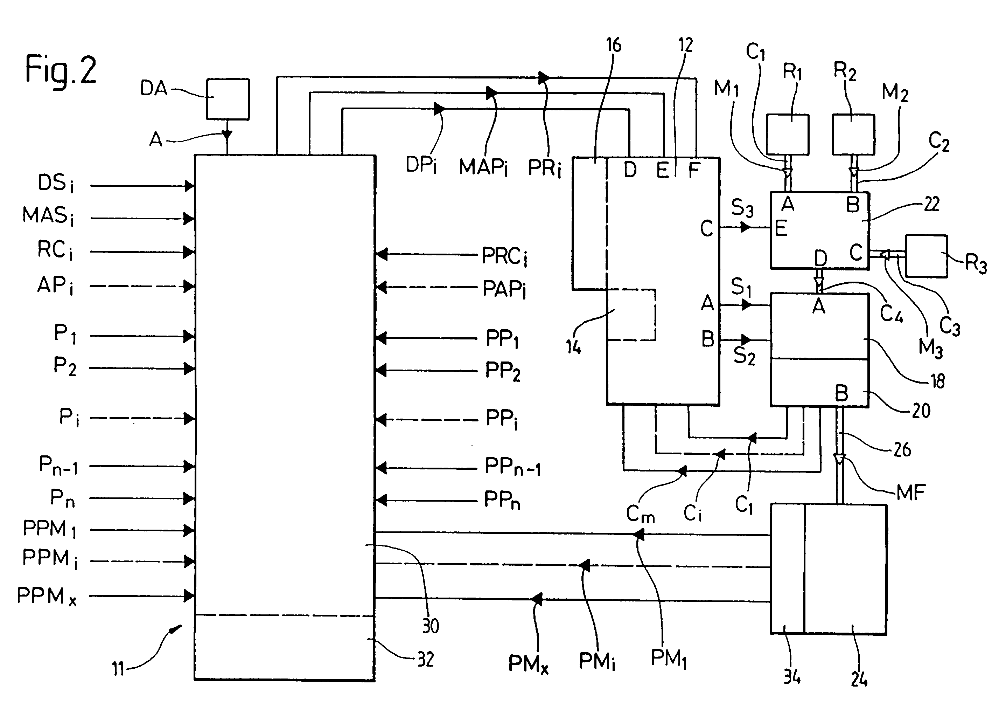

En se référant à la figure 2, on décrira ci-après un deuxième mode de réalisation de l'invention.

Ce deuxième mode de réalisation de l'invention concerne un dispositif d'administration de plusieurs médicaments M₁, M₂ à M₃, ce dispositif d'administration de médicaments comprenant un système d'aide à la décision 11. En outre, ce dispositif d'administration comprend une pompe 18, un circuit de commande 12 de cette pompe 18 associé à une base de données 16. Le circuit de commande 12 comprend une horloge interne 14. La pompe 18 est associée à une unité multicapteur 20.

Il est également prévu un sélecteur 22 ayant ses entrées A, B et C respectivement reliées à trois réservoirs R₁, R₂ et R₃ au moyen de conduits C₁, C₂ et C₃, et sa sortie D reliée à une entrée A de la pompe 18 au moyen d'un conduit C₄. La sortie B de l'unité multicapteur 20 est destinée à être reliée à un être vivant représenté schématiquement par un bloc 24 à l'aide d'un conduit 26.

On remarquera que la pompe 18 et l'unité multicapteur 20 peuvent former ensemble une seule unité, les capteurs prévus dans l'unité multicapteur 20 n'étant pas nécessairement situés en aval de la pompe 18 relativement à l'écoulement du médicament fluide MF. De même, le sélecteur 22 peut être directement associé à la pompe 18 sans qu'il y ait lieu de prévoir un conduit C₄ de raccord entre le sélecteur 22 et la pompe 18.

Dans le présent mode de réalisation, il est prévu par exemple une pompe 18 formée par une micropompe actionnée par un élément piézo-électrique. Le circuit de commande 12 fournit à ses sorties A et B respectivement deux signaux de commande S₁ et S₂ dont les valeurs sont représentatives d'une fréquence et d'une amplitude d'actionnement de la micropompe 18 au moyen de l'élément piézo-électrique (non-représenté). Ces signaux de commande S₁ et S₂ pourront être combinés en un seul signal S₁,₂ et prendre d'autres significations que fréquence et amplitude selon le type de pompe à actionner. De plus, le circuit de commande 12 fournit à sa sortie C un troisième signal de commande S₃ transmis à une entrée E du sélecteur 22. L'unité multicapteur 20 fournit au circuit de commande 12 plusieurs signaux de contrôle Cᵢ, i = 1 à m. Les signaux de contrôle Cᵢ sont par exemple représentatifs de la pression à la sortie de la micropompe 18, de la température du médicament MF, du débit volumique ou massique de ce médicament MF fourni par cette micropompe.

Dans un mode de réalisation particulier, le circuit de commande 12 comprend un module de commande à logique floue (non-représenté)et les différents signaux de contrôle Cᵢ sont transformés en variables floues traitées par ce module de commande à logique floue.

On remarquera que le circuit de commande 12 peut comporter d'autres sorties non-représentées et fournir d'autres signaux de commande à la pompe 18, au sélecteur 22 et même aux réservoirs R₁, R₂, R₃ ou à des éléments de contrôle associés à ces réservoirs tels que des vannes du type ouvert/fermé ou anti-retour.

Le système d'aide à la décision 11 du dispositif d'administration représenté à la figure 2 comprend une unité de traitement à logique floue 30 associée à une base de données 32. Cette unité de traitement à logique floue 30 est agencée pour recevoir en entrée plusieurs signaux d'informations concernant chacun des médicaments M₁ à M₃ et l'être vivant 24 auquel ces médicaments sont destinés. Parmi les informations concernant les médicaments Mᵢ, i = 1 à 3, il est prévu de fournir en entrée pour chacun de ces médicaments la dose standard DSᵢ, le mode d'administration standard MASᵢ, le rythme circadien RCᵢ pour au moins un organe ou une partie du corps concerné par le médicament Mᵢ, ainsi que d'autres informations ou paramètre APᵢ relatifs aux médicaments Mᵢ, i = 1 à 3.

Parmi ces autres informations fournies à l'unité de traitement à logique floue 30, on mentionnera par exemple l'organe ou la partie du corps auquel est destiné le médicament Mᵢ, la composition chimique de ce médicament Mᵢ ou encore d'autres informations relatives aux effets secondaires connus pour ce médicament Mᵢ, des incompatibilités avec d'autres médicaments, la quantité maximum administrable à un être vivant particulier et toutes autres informations susceptibles d'influencer la quantité personnalisée DPᵢ de chaque médicament Mᵢ ou son mode d'administration personnalisé MAPᵢ fourni par l'unité de traitement à logique floue 30 respectivement aux entrées D et E du circuit de commande 12.

Les informations susceptibles d'influencer la quantité personnalisée DPi de chaque médicaments Mᵢ ou son mode d'aministration personnalisé MAPi sont notamment en relation avec les diverses informations fournies à l'unité de traitement 30 concernant le patient auquel est destinée la thérapie. Ces informations déterminent par exemple la valeur de coefficients ou paramètres introduits dans les diverses règles d'inférence prévues dans l'unité de traitement 30 et/ou dans les équations d'agrégations des résultats de ces diverses règles d'inférence.

Les informations, fournies en entrée à l'unité de traitement 30, concernant l'être vivant, auquel les médicaments Mᵢ, i = 1 à 3 sont destinés, sont désignées sur la figure 2 par les références P₁ à Pₙ. Ces informations Pᵢ, i = 1 à n relatives au patient peuvent comporter les mêmes informations que celles décrites ci-avant dans le premier mode de réalisation de l'invention. De plus, il est possible de prévoir des informations concernant des habitudes temporelles de l'être vivant considéré ici et des informations temporelles concernant des organes ou parties corporelles de cet être vivant. De même, il est possible d'introduire des paramètres relatifs à l'état psychique du patient lorsque les médicaments à administrer ont une corrélation avec de telles informations.

On mentionnera ici encore comme informations d'entrée susceptibles d'intervenir dans la détermination des quantités personnalisées DPᵢ et des modes d'administration personnalisés MAPᵢ de ces quantités de médicaments un rythme circadien général ou un biorythme global de l'être vivant auquel est destiné le dispositif d'administration de médicament décrit ici.

Concernant les informations relatives au mode d'administration standard MASᵢ pour chaque médicament Mᵢ, il s'agira par exemple d'un nombre de doses à administrer sur une journée ou sur un cycle circadien. L'information relative au mode d'administration peut également concerner un débit maximum ou une courbe caractéristique pour le débit lors de l'administration d'une dose. Dans le cas d'une administration en continu ou en quasi-continu, le mode d'administration MASᵢ indique le débit standard sous lequel le médicament doit être administré ou alors le taux de répétition standard d'administration de microdoses. Il est possible de prévoir dans la base de données 32 un ensemble de courbes de débit caractéristiques pouvant être sélectionnées par l'utilisateur du dispositif d'administration selon l'invention.

Pour ce qui concerne le rythme circadien RCᵢ, on prendra par exemple une variable floue dont le référentiel est divisé en quatre zones floues représentant quatre plages temporelles 20-22, 22-24, 24-26 et 26-28 heures.

On remarquera que l'unité de traitement à logique floue 30 peut traiter dans un premier temps un certain nombre de variables floues, puis transformer certains résultats de ce traitement sous forme de variables définies pouvant être ensuite traitées de manière classique avec d'autres variables d'entrée. Les variables traitées comme variables floues peuvent être entrées dans le système d'aide à la décision comme telles ou être entrées sous forme de variables définies transformées ensuite en variables floues par l'unité de traitement à logique floue 30.

Dans ce deuxième mode de réalisation de l'invention, il est également prévu en entrée du système d'aide à la décision 11 des signaux de mesures PMᵢ, i = 1 à x, provenant de capteurs 34 associés à l'être vivant 24 auquel est associé le dispositif d'administration selon l'invention. Les signaux de mesures PMᵢ constituent donc des informations variables dans le temps donnant des indications sur l'évolution de certains paramètres propres à l'être vivant 24. Les signaux de mesures PMᵢ sont transformés en variables floues par l'unité de traitement à logique floue 30.

A titres d'exemples, les signaux de mesures PMᵢ sont représentatifs de la pression sanguine, de la température et de la concentration d'une substance dans le sang ou dans une autre partie du corps. Dans une application particulière, un ou plusieurs signaux de mesures PMᵢ peuvent provenir d'une électromyographie ou de tout autre dispositif de surveillance de l'être vivant, par exemple un électrocardiogramme.

Dans ce mode de réalisation, il est également prévu que le système d'aide à la décision 11 fournisse au circuit de commande 12 un signal PRᵢ représentatif d'un degré de priorité de l'administration du médicament Mᵢ, i = 1 à 3. Ce degré de priorité permet au circuit de commande 12 de gérer de manière optimale l'administration des divers médicaments Mᵢ lorsque l'administration de plusieurs médicaments interviennent au même moment.

On notera que l'information du degré de priorité PRᵢ de l'administration des divers médicaments à sa raison d'être spécialement lorsque la pompe 18 est agencée pour fournir un débit maximum inférieur à la somme des débits maximum prévus pour les médicaments Mᵢ. De plus, les degrés de priorité PRᵢ, fournis à l'entrée F du circuit de commande 12, peuvent être également traités par un module de commande à logique floue (non-représenté) compris dans le circuit de commande 12 pour déterminer le fonctionnement optimal de la pompe 18 de manière à minimiser la consommation en énergie du dispositif d'administration selon l'invention.

Concernant le fonctionnement du dispositif d'administration selon l'invention, il est prévu que la base de données 16 comporte les caractéristiques de consommation en énergie de la pompe 18 relativement à divers paramètres propres à cette pompe 18. Dans le cas particulier d'une micropompe actionnée par un élément piézo-électrique, les paramètres déterminants de la consommation sont l'amplitude et la fréquence du signal électrique d'excitation de l'élément piézo-électrique. Ces paramètres caractéristiques déterminent le débit de médicament MF sortant de la micropompe 18.

Le module de commande à logique floue (non-représenté) compris dans le circuit de commande 12 traite les informations provenant du système d'aide à la décision 11 en association avec les paramètres propres à la pompe 18 de manière à administrer les divers médicaments Mᵢ, i = 1 à 3, avec un rendement énergétique optimal, c'est-à-dire en minimisant au mieux la consommation d'énergie tout en garantissant une efficacité thérapeutique et la sécurité requise dans l'administration des divers médicaments Mᵢ.

On notera encore que les signaux de contrôle Cᵢ fournis au circuit de commande 12 par l'unité multicapteur 20 peuvent également être traités par le module de commande à logique floue susmentionné pour intervenir dans la détermination des divers signaux de commande S₁, S₂ et S₃ fournis par le circuit de commande 12 à la pompe 18 et au sélecteur 22 et/ou assurer le réglage de ces signaux de commande.

Etant donné que le système d'aide à la décision 11 reçoit en entrée diverses informations variables dans le temps provenant des capteurs 34 associés à l'être vivant 24, les signaux d'informations DPᵢ, MAPᵢ et PRᵢ fournis par ce système d'aide à la décision 11 au circuit de commande 12 sont susceptibles de varier au cours du temps.

On notera aussi que dans une variante de réalisation équivalente, les paramètres caractéristiques de la pompe 18 peuvent être introduits dans la base de données 32 associée à l'unité de traitement à logique floue 30 du système d'aide à la décision 11. Dans ce cas, l'unité de traitement 30 traite ces paramètres caractéristiques de la pompe 18 et de son fonctionnement à rendement optimal conjointement avec les autres informations fournies en entrée de ce système d'aide à la décision 11. Ainsi, le système d'aide à la décision 11 fournit un signal pour les quantités de médicaments personnalisées DPᵢ et pour les modes d'administration personnalisés MAPᵢ de ces médicaments compatibles avec le fonctionnement et les capacités de pompage de la pompe 18. Dans cette variante de réalisation, l'information concernant les degrés de priorité PRᵢ dans l'administration des divers médicaments n'a plus de raison d'être étant donné que cette information est déjà intégrée dans le signal d'information concernant le mode d'administration personnalisé MAPᵢ de chaque médicament Mᵢ.

On remarquera également que l'information relative au mode d'administration personnalisé MAPᵢ peut également contenir l'information relative à la quantité de médicaments personnalisée DPᵢ. Dans ce cas particulier, les signaux DPᵢ et MAPᵢ représentés sur la figure 2 sont confondus.

Il découle plusieurs avantages du deuxième mode de réalisation de l'invention, notamment le fait que non seulement les quantités de médicaments à administrer sont personnalisées, mais également le mode d'administration dans le temps de ces quantités de médicaments.

Le mode d'administration dans le temps de chaque médicament prévu est déterminé en fonction d'au moins certaines informations reçues en entrée par le système d'aide à la décision 11. Ainsi, l'administration dans le temps de chaque médicament est personnalisée, ce qui rend le traitement thérapeutique efficace et permet également de minimiser les quantités de médicaments à administrer à l'être vivant auquel la thérapie est destinée. De plus, en assurant une gestion personnalisée de l'administration du ou des médicament(s), on minimise le risque d'effets secondaires néfastes à l'être vivant considéré.

Le système d'aide à la décision selon l'invention est souple et permet une adaptation du traitement thérapeutique en variant les diverses informations relatives à l'être vivant selon l'évolution de ces diverses informations au cours du traitement thérapeutique.

Dans le deuxième mode de réalisation décrit ici, il est également prévu de fournir à l'unité de traitement à logique floue 30 des informations concernant les degrés d'importance ou de pertinence des informations relatives aux médicaments et des informations relatives à l'être vivant auquel ces médicaments sont destinés. Ces degrés d'importance ou de pertinence sont considérés par l'unité de traitement 30 comme des facteurs de pondération pris en considération dans le traitement des diverses variables effectué par l'unité de traitement 30. A cet effet, il est prévu en entrée de cette unité de traitement 30 un signal PRCᵢ de pondération du rythme circadien, un signal PAPᵢ de pondération de l'information APᵢ décrite ci-avant, des signaux PPᵢ, i = 1 à n, associés aux informations Pᵢ, i = 1 à n, décrits ci-avant, ainsi que des signaux PPMᵢ, i = 1 à x, constituant des facteurs de pondération des signaux de mesures PMᵢ fournis par les capteurs 34.

Les facteurs de pondération permettent ainsi de donner un poids différent à chacune des informations d'entrée fournies au système d'aide à la décision 11. Cette caractéristique confère une très grande flexibilité au système d'aide à la décision 11.

Les informations d'entrée relatives aux médicaments, tout comme les différents facteurs de pondération mentionnés ci-avant peuvent être introduits dans le système d'aide à la décision 11 de diverses manières, notamment à l'aide d'un support informatique quelconque.

Pour chaque médicament ou pour chaque combinaison de médicaments, il est possible de prévoir autant de modules appropriés contenant les informations nécessaires à fournir au système d'aide à la décision 11. Ainsi, pour chaque traitement spécifique pour lequel le système d'aide à la décision 11 est utilisé, il sera possible, à l'aide d'une interface approprié, de transmettre ces données à la base de données 32 prévues en association avec l'unité de traitement 30.

On notera que la base de données 32 peut comprendre toutes sortes d'informations utiles, notamment concernant diverses parties et organes du corps humain, les diverses substances constituant un répertoire de médicaments, des données concernant divers effets secondaires et/ou diverses incompatibilités entre certains médicaments. Il est également sans autre possible de stocker dans cette base de données 32 l'ensemble des informations fournies en entrée du système d'aide à la décision 11.

Dans une variante encore plus évoluée, il est possible d'agencer l'unité de traitement 30 et la base de données 32 de manière à former un système expert ayant une base de connaissances évolutive, capable d'intégrer diverses informations et conclusions apportées lors d'utilisations antérieures du système d'aide à la décision 11.

Il est possible de prévoir un dispositif d'alarme DA agencé pour fournir en cas de besoin un signal d'alarme A à l'unité de traitement 30. Dans une variante de réalisation, le signal d'alarme A peut être fourni directement au circuit de commande 12. Ainsi, il est possible à tout moment de fournir une dose d'urgence d'un médicament ou de plusieurs médicaments si cela s'avère nécessaire. La quantité de médicaments et le mode d'administration de cette dose d'urgence peut être également déterminée par le système d'aide à la décision 11.

On notera encore que le système d'aide à la décision 11 peut former une unité physiquement indépendante de l'ensemble formé par la pompe 18 et le circuit de commande 12 de cette pompe. Dans ce cas, il est prévu une interface entre le système d'aide à la décision 11 et le circuit de commande 12 permettant de fournir à ce circuit de commande les signaux d'informations prévus avant le début du traitement thérapeutique, ou de fournir ces signaux d'informations avec une fréquence temporelle déterminée ou encore de manière continue tout au long d'un traitement thérapeutique.

Dans le cas où toutes les informations d'entrée du système d'aide à la décision 11 sont constantes, il est sans autre possible que le système système d'aide à la décision 11 forme une unité indépendante pouvant se trouver par exemple chez un médecin traitant, alors que la pompe 18 et le circuit de commande 12 et l'ensemble des autres unités liées à la pompe 18 sont portées durant un traitement thérapeutique par le patient qui y est soumis. Par contre, lorsque le système d'aide à la décision 11 reçoit des informations en temps réel provenant de capteurs 34 associés au patient, il est nécessaire que le système d'aide à la décision 11 soit relié au circuit de commande 12 au moins périodiquement.

Finalement, on remarquera que le dispositif d'administration d'un médicament selon l'invention ne se limite pas au cas décrit ci-avant dans lequel il est prévu une pompe (18) pour l'administration d'un médicament fluide (MF) , mais à tout dispositif susceptible d'administrer ou de participer à l'administration d'un ou plusieurs médicament(s) au moyen d'une ou plusieurs pompe(s) de manière simultanée et/ou séquentielle.

## Revendications

1. Système d'aide à la décision (1;11), pour l'administration d'au moins un médicament à un être vivant, comprenant des moyens d'introduction de données permettant à un utilisateur d'introduire un premier groupe d'informations (P₁ à Pₙ) relatives audit être vivant et un deuxième groupe d'informations (PP₁ à PPₙ) constitué par des facteurs de pondération associés respectivement auxdites informations dudit premier groupe d'informations, ce système d'aide à la décision comprenant en outre une unité de traitement à logique floue (8;30) agencée pour traiter au moins ledit premier groupe d'informations et ledit deuxième groupe d'informations et pour fournir au moins un signal de sortie (DP;DPᵢ,MAPᵢ) représentatif d'une quantité dudit médicament à administrer audit être vivant et dépendant dudit premier groupe d'informations et dudit deuxième groupe d'informations.

2. Système d'aide à la décision selon la revendication 1, dans lequel lesdits moyens d'introduction de données permettent audit utilisateur d'introduire au moins une information (DSᵢ,MASᵢ,RCᵢ,APᵢ) relative audit médicament, ladite unité de traitement à logique floue (8;30) étant agencée pour traiter l'information relative audit médicament et ledit signal de sortie dépendant en outre de cette information.

3. Système d'aide à la décision selon la revendication 2, dans lequel lesdits moyens d'introduction de données permettent audit utilisateur d'introduire un troisième groupe d'informations relatives audit médicament et un quatrième groupe d'informations (PRCᵢ, PAPᵢ) constitué par des facteurs de pondération associés respectivement auxdites informations dudit troisième groupe d'informations, ce système d'aide à la décision fournissant au moins un signal de sortie dépendant desdits troisième et quatrième groupes d'informations.

4. Système d'aide à la décision selon la revendication 3, dans lequel ledit troisième groupe d'informations comprend au moins une information relative à une quantité standard (DSᵢ) dudit médicament et au moins une information (MASᵢ) relative à un mode d'administration dans le temps dudit médicament, ce système d'aide à la décision fournissant en sortie au moins une information relative à une quantité personnalisée (DPᵢ) dudit médicament à administrer audit être vivant et au moins une information (MAPᵢ) relative à un mode d'administration personnalisé dans le temps de ce médicament à cet être vivant.

5. Système d'aide à la décision selon la revendication 4, dans lequel ladite unité de traitement à logique floue est agencée pour traiter en outre au moins une information relative au biorythme d'un organe de l'être vivant auquel est destiné ledit médicament.

6. Système d'aide à la décision selon la revendication 4 ou 5, dans lequel ladite unité de traitement à logique floue est agencée pour traiter en outre au moins une information relative au rythme circadien de l'être vivant auquel est destiné ledit médicament ou au rythme circadien d'un organe de cet être vivant auquel est destiné ledit médicament.

7. Système d'aide à la décision selon l'une des revendications précédentes, agencé pour traiter conjointement des informations relatives à plusieurs médicaments différents (M₁,M₂,M₃), ce système d'aide à la décision fournissant en sortie, pour chacun desdits médicaments différents, au moins une information (DPᵢ, i = 1,2,3) représentative d'une quantité de ce médicament, cette information dépendant d'au moins une information (P₁ à Pₙ) relative audit être vivant fournie par un utilisateur à ladite unité de traitement à logique floue (30) par l'intermédiaire desdits moyens d'introduction de données.

8. Système d'aide à la décision, selon l'une des revendications précédentes, caractérisé en ce que ladite unité de traitement à logique floue (30) est associée à une base de données complémentaires (32) comprenant diverses données sur au moins un médicament et/ou un organe auquel ce médicament est destiné.

9. Dispositif d'administration d'au moins un médicament (M₁,M₂,M₃) à un être vivant (24) comprenant des moyens (18) d'administration d'au moins un médicament et un circuit de commande (12) de ces moyens, ce dispositif étant caractérisé en ce qu'il comprend en outre un système d'aide à la décision (1;11) selon l'une des revendications 1 à 8, ce système d'aide à la décision fournissant ledit signal de sortie (DP;DPᵢ,MAPᵢ) audit circuit de commande.

10. Dispositif d'administration selon la revendication 9, caractérisé en ce qu'il comporte en outre au moins un capteur (20) prévu en sortie desdits moyens (18) d'administration d'au moins un médicament, ce capteur fournissant audit circuit de commande (12) un signal de contrôle (Cᵢ, i = 1 à m) servant au réglage d'au moins un signal de commande (S₁,S₂,S₃) fourni par ce circuit de commande à ladite pompe.

11. Dispositif d'administration selon la revendication 9 ou 10, caractérisé en ce que lesdits moyens (18) d'administration d'au moins un médicament sont agencés pour administrer sélectivement plusieurs médicaments (M₁,M₂,M₃) audit être vivant (24).

12. Dispositif d'administration, selon la revendication 11, caractérisé en ce que ledit système d'aide à la décision (11) est agencé pour fournir audit circuit de commande (12) des signaux de priorité relatifs auxdits plusieurs médicaments (M₁,M₂,M₃), ledit circuit de commande comportant un module de commande à logique floue agencé pour traiter lesdits signaux de priorité en fonction de caractéristiques propres auxdits moyens d'administration et des informations reçues par ledit circuit de commande, relatives au mode d'administration personnalisé (MAPᵢ, i = 1,2,3) dans le temps de chacun desdits plusieurs médicaments, ledit circuit de commande fournissant au moins un signal de commande (S₁;S₂) auxdits moyens d'administration (18) dépendant desdits signaux de priorité.

13. Dispositif d'administration selon la revendication 12, caractérisé en ce qu'il comporte plusieurs réservoirs (R₁,R₂,R₃) prévus respectivement pour lesdits plusieurs médicaments (M₁,M₂,M₃), ce dispositif d'administration comportant en outre un sélecteur (22) comprenant plusieurs entrées (22A,22B,22C) reliées respectivement auxdits plusieurs réservoirs par des conduits d'entrée (C₁,C₂,C₃) et une sortie (22D) reliées auxdits moyens d'administration (18) par un conduit de sortie (C₄), ledit circuit de commande (12) étant agencé pour commander ledit sélecteur conjointement auxdits moyens d'administration.

14. Dispositif d'administration selon l'une des revendications 9 à 13, caractérisé en ce que ledit circuit de commande (12) est associé à une base de données (16) relative aux caractéristiques de consommation en énergie desdits moyens (18) d'administration d'au moins un médicament, ce circuit de commande comprenant un module de commande à logique floue permettant de commander ces moyens d'administration avec un rendement énergétique optimal.

15. Dispositif d'administration selon l'une quelconque des revendications 9 à 14, caractérisé en ce qu'il comprend un dispositif d'alarme (DA) permettant l'administration d'une dose d'urgence audit être vivant (24) relié auxdits moyens (18) d'administration d'au moins un médicament.

## Patentansprüche

1. Entscheidungsunterstützungssystem (1; 11) zur Verabreichung mindestens eines Medikaments an ein Lebewesen, umfassend Mittel zur Dateneingabe, welche es einem Benutzer gestatten, eine erste Gruppe von Informationen (P₁ bis Pₙ) über das Lebewesen sowie eine zweite Gruppe von Informationen (PP₁ bis PPₙ), bestehend aus den Informationen der ersten Gruppe von Informationen entsprechenden Gewichtungsfaktoren, einzugeben, wobei das Entscheidungsunterstützungssystem zudem eine Fuzzy-Logik-Verarbeitungseinheit (8; 30) umfaßt, vorgesehen zur Verarbeitung mindestens der ersten Gruppe von Informationen und der zweiten Gruppe von Informationen, um mindestens ein Ausgangssignal (DP; DPᵢ, MAPᵢ) zu erzeugen, welches einer Menge des dem Lebewesen zu verabreichenden Medikaments entspricht und von der ersten Gruppe von Informationen und der zweiten Gruppe von Informationen abhängt.

2. Entscheidungsunterstützungssystem nach Anspruch 1, in welchem die Mittel zur Dateneingabe dem Benutzer die Eingabe mindestens einer Information (DSᵢ, MASᵢ, RCᵢ, APᵢ) über das Medikament gestatten, wobei die Fuzzy-Logik-Verarbeitungseinheit (8; 30) zur Verarbeitung der Information über das Medikament ausgebildet ist und das Ausgangssignal unter anderem von dieser Information abhängt.

3. Entscheidungsunterstützungssystem nach Anspruch 2, in welchem die Mittel zur Dateneingabe dem Benutzer die Eingabe einer dritten Gruppe von Informationen über das Medikament und einer vierten Gruppe von Informationen (PRCᵢ, PAPᵢ), bestehend aus den Informationen der dritten Gruppe entsprechenden Gewichtungsfaktoren, gestatten, wobei das Entscheidungsunterstützungssystem mindestens ein von der dritten und vierten Gruppe von Informationen abhängiges Ausgangssignal erzeugt.

4. Entscheidungsunterstützungssystem nach Anspruch 3, in welchem die dritte Gruppe von Informationen mindestens eine Information über eine Standard menge (DSᵢ) des Medikaments und mindestens eine Information (MASᵢ) über eine zeitliche Verabreichungsweise des Medikaments umfaßt, wobei das Entscheidungsunterstützungssystem am Ausgang mindestens eine Information über eine personalisierte Menge (DPᵢ) des an das Lebewesen zu verabreichenden Medikaments und mindestens eine Information (MAPᵢ) über eine personalisierte zeitliche Verabreichungsweise des Medikaments an das Lebewesen bereitstellt.

5. Entscheidungsunterstützungssystem nach Anspruch 4, in welchem die Fuzzy-Logik-Verarbeitungseinheit ausgebildet ist, um zudem mindestens eine Information über den Biorythmus eines Organs des Lebewesens, für welches das Medikament bestimmt ist, zu verarbeiten.

6. Entscheidungsunterstützungssystem nach Anspruch 4 oder 5, in welchem die Fuzzy-Logik-Verarbeitungseinheit zur Verarbeitung unter anderem mindestens einer Information über den 24-Stunden-Rhythmus des Lebewesens, für welches das Medikament vorgesehen ist, oder den 24-Stunden-Rhythmus eines Organs des Lebewesens, für welches das Medikament vorgesehen ist, ausgebildet ist.

7. Entscheidungsunterstützungssystem nach einem der vorangehenden Ansprüche, ausgebildet zur gemeinsamen Verarbeitung von Informationen über mehrere verschiedene Medikamente (M₁, M₂, M₃), wobei das Entscheidungsunterstützungssystem am Ausgang für jedes der verschiedenen Medikamente mindestens eine Information (DPᵢ, i=1, 2, 3) über eine Größe dieses Medikaments bereitstellt, wobei diese Information von mindestens einer von einem Benutzer der Fuzzy-Logik-Verarbeitungseinheit (30) mittels der Mittel zur Dateneingabe bereitgestellten Information (P₁ bis Pₙ) über das Lebewesen abhängt.

8. Entscheidungsunterstützungssystem nach einem der vorangehenden Ansprüche, dadurch gekennzeichnet, daß die Fuzzy-Logik-Verarbeitungseinheit (30) mit einer ergänzenden, verschiedene Daten über mindestens ein Medikament und/oder Organ, für welches das Medikament bestimmt ist, umfassenden Datenbank (32) verbunden ist.

9. Vorrichtung zur Verabreichung mindestens eines Medikaments (M₁, M₂, M₃) an ein Lebewesen (24), umfassend Mittel (18) zur Verabreichung mindestens eines Medikaments und einen Schaltkreis (12) zur Steuerung dieser Mittel, wobei diese Vorrichtung dadurch gekennzeichnet ist, daß sie zudem ein Entscheidungsunterstützungssystem (1; 11) nach einem der Ansprüche 1 bis 8 umfaßt, wobei dieses Entscheidungsunterstützungssystem das Ausgangssignal (DP; DPᵢ, MAPᵢ) am Schaltkreis zur Steuerung bereitstellt.

10. Vorrichtung zur Verabreichung nach Anspruch 9, dadurch gekennzeichnet, daß sie zudem mindestens einen am Ausgang der Mittel (18) zur Verabreichung mindestens eines Medikaments vorgesehenen Meßgrößenaufnehmer (20) umfaßt, wobei der Meßgrößenaufnehmer am Schaltkreis (12) zur Steuerung ein Steuersignal (Cᵢ, i= 1, 2, 3) bereitstellt, welches der Steuerung mindestens eines durch den Schaltkreis zur Steuerung an die Pumpe angelegten Steuersignals (S₁, S₂, S₃) dient.

11. Vorrichtung zur Verabreichung nach Anspruch 9 oder 10, dadurch gekennzeichnet, daß die Mittel (18) zur Verabreichung mindestens eines Medikaments zur selektiven Verabreichung mehrerer Medikamente an das Lebewesen (24) ausgebildet sind.

12. Vorrichtung zur Verabreichung nach Anspruch 11, dadurch gekennzeichnet, daß das Entscheidungsunterstützungssystem (11) zur Bereitstellung von den mehreren Medikamenten (M₁, M₂, M₃) zugeordneten Prioritätssignalen am Schaltkreis (12) zur Steuerung ausgebildet ist, wobei der Schaltkreis zur Steuerung ein Steuermodul mit Fuzzy-Logik umfaßt, welches ausgebildet ist, die Prioritätssignale abhängig von den eigentlichen Eigenschaften der Mittel zur Verabreichung und von durch den Schaltkreis zur Steuerung empfangenen Informationen über die personalisierte zeitliche Verabreichungsweise (MAPᵢ, i=1, 2, 3) jedes der mehreren Medikamente zu verarbeiten, wobei der Schaltkreis zur Steuerung abhängig von den Prioritätssignalen mindestens ein Steuersignal (S₁; S₂) an die Mittel (18) zur Verabreichung liefert.

13. Vorrichtung zur Verabreichung nach Anspruch 17, dadurch gekennzeichnet, daß sie mehrere für die entsprechenden Medikamente (M₁, M₂, M₃) vorgesehene Behälter (R₁, R₂, R₃) umfaßt, wobei die Vorrichtung zur Verabreichung zudem eine Auswahleinrichtung (22) umfaßt, welche mehrere entsprechend durch Eingangsleitungen (C₁, C₂, C₃) mit den mehreren Behältern verbundene Eingänge (22A, 22B, 22C) und einen durch eine Ausgangsleitung (C₄) mit den Mitteln (18) zur Verabreichung verbundenen Ausgang (22D) umfaßt, wobei der Schaltkreis (12) zur Steuerung ausgebildet ist, die Auswahlvorrichtung gemeinsam mit den Mitteln zur Verabreichung zu steuern.

14. Vorrichtung zur Verabreichung nach einem der Ansprüche 9 bis 13, dadurch gekennzeichnet, daß der Schaltkreis (12) zur Steuerung mit einer Datenbank (16) über charakteristische Energieverbrauchsdaten der Mittel (18) zur Verabreichung mindestens eines Medikaments verbunden ist, wobei der Schaltkreis zur Steuerung ein Steuermodul mit Fuzzy-Logik umfaßt, welches es ermöglicht, die Mittel zur Verabreichung mit einer optimalen Energienutzung zu steuern.

15. Vorrichtung zur Verabreichung nach einem der Ansprüche 9 bis 14, dadurch gekennzeichnet, daß sie eine Alarmeinrichtung (DA) umfaßt, welche die Verabreichung einer Noffalldosis an das mit den Mitteln (18) zur Verabreichung mindestens eines Medikaments verbundene Lebewesen (24) ermöglicht.

## Claims

1. Decision making aid system (1; 11), for delivering at least one drug to a living being, comprising data introduction means allowing a user to introduce a first group of information (P₁ to Pₙ) relative to said living being and a second group of information (PP₁ to PPₙ) constituted by weighting factors respectively associated to said information of said first group of information, this decision making aid system further comprising a fuzzy logic processing unit (8; 30) arranged to process at least said first group of information and said second group of information and for providing at least an output signal (DP; DPᵢ, MAPᵢ) representative of the quantity of said drug to be delivered to said living being and depending on said first group of information and on second said second group of information.

2. Decision making aid system according to claim 1, wherein said data introduction means allow said user to introduce at least one information (DSᵢ, MASᵢ, RCᵢ,APᵢ) relative to said drug, said fuzzy logic processing unit (8, 30) being arranged to process the information relative to said drug and said output signal also depending on this information.

3. Decision making aid system according to claim 2, wherein said data introduction means allow said user to introduce a third group of information relative to said drug and a fourth group of information (PRCᵢ, PAPᵢ) constituted by weighting factors respectively associated with said information of said third group of information, this decision making aid system providing at least an output signal depending on said third and said fourth group of information.

4. Decision making aid system according to claim 3, wherein said third group of information comprises at least an information relative to a standard quantity (DSᵢ) of said drug and at least an information (MASᵢ) relative to a time delivering mode of said drug, the decision making aid system providing at its output at least an information relative to a personalised quantity (DPᵢ) of said drug to be delivered to said living being and at least an information (MAPᵢ) relative to a personalised time delivering mode of this drug to this living being.

5. Decision making aid system according to claim 4, wherein said fuzzy logic processing unit is arranged for processing at least an information relative to the biorhythm of an organ of a living being for whom the drug is intended.

6. Decision making aid system according to claim 4 or 5, wherein said fuzzy logic processing unit is arranged to further process at least an information relative to the circadian rhythm of said living being for whom said drug is intended or the circadian rhythm of an organ of this living being for whom said drug is intended.

7. Decision making aid system according to any one of the preceding claims, arranged to jointly process the information relative to several different drugs (M₁, M₂, M₃), this decision making aid system providing at its output, for each of said different drugs at least an information (DPᵢ, i = 1,2,3) representative of a quantity of this drug, this information depending at least on an information (P₁ to Pₙ) relative to said living being provided by a user to said fuzzy logic processing unit (30) via said data introduction means.

8. Decision making aid system according to any one of the preceding claims, characterised in that said fuzzy logic processing unit (30) is associated with a complementary data base (32) comprising several data on at least a drug and/or an organ for which this drug is intended.

9. Device for delivering at least one drug (M₁, M₂, M₃) to a living being (24) comprising delivering means (18) of at least a drug and a control circuit (12) of this means, this device is being characterised in that it further comprises a decision making aid system (1;11) according to any one of claims 1 to 8, this decision making aid system providing said output signal (SP; Dpᵢ, MAPᵢ) to said control circuit.

10. Device for delivering according to claim 9, characterised in that it further comprises at least a sensor (20) provided at the output of said means for delivering at least a drug, this sensor providing to said control circuit (12) a check signal (Cᵢ, i = 1 to m) serving to adjust at least a control signal (S₁, S₂, S₃) provided by this control circuit to said pump.

11. Device for delivering according to claim 9 or 10, characterised in that said means (18) for delivering at least a drug is arranged to selectively deliver several drugs (M₁, M₂, M₃) to said living being (24).

12. Device for delivering according to claim 11, characterised in that said decision making aid system (11) is arranged to provide to said control circuit (12) priority signals relative to said several drugs (M₁, M₂, M₃), said control circuit comprising a fuzzy logic control module arranged to process said priority signals as a function of features proper to said means for delivering and information received by said control circuit, relative to a personalised time delivery mode (MAPᵢ, i = 1, 2, 3) of each of said several drugs, said control circuit providing at least a control signal (S₁; S₂) to said means for delivering (18) depending on said priority signals.

13. Device for delivering according to claim 12, characterised in that it comprises several reservoirs (R₁, R₂, R₃) provided for said several drugs (M₁, M₂, M₃) respectively, this device for delivering further comprising a selector (22) comprising several input (22A, 22B, 22C) respectively connected to said several reservoirs by input channels (C₁, C₂, C₃) and an output (22D) connected to said means for delivering (18) by an output channel (C₄), said control circuit (12) being arranged to control said selector together with said means for delivering.

14. Device for delivering according to anyone of claims 9 to 13, characterised in that said control circuit (12) is associated with a database (16) relative to energy consumption features of said means for delivering (18) of at least a drug, this control circuit comprising a fuzzy logic control module allowing to control this means for delivering with an optimised energetic yield.

15. Device for delivering according to anyone of claims 9 to 14, characterised in that it comprises an alarm device (DA) allowing the delivery of an emergency dose to said living being (24) connected to said means for delivering (18) at least one drug.
